# EUROPÄISCHE PATENTSCHRIFT

(11) **EP 4 389 021 B1**
(45) Veröffentlichungstag und Bekanntmachung des Hinweises auf die Patenterteilung: **06.05.2026**
(21) Anmeldenummer: 23216151.3
(22) Anmeldetag: 13.12.2023
(51) Int. Cl.: A61B 17/16

(54) **CHIRURGISCHES FRÄSWERKZEUG MIT OPTIMIERTER GEOMETRIE**
SURGICAL MILLING TOOL WITH OPTIMIZED GEOMETRY
FRAISE CHIRURGICALE À GÉOMÉTRIE OPTIMISÉE

(30) Priorität: 20.12.2022 DE 102022134173
(43) Veröffentlichungstag der Anmeldung: 26.06.2024
(73) Patentinhaber: Aesculap AG, 78532 Tuttlingen (DE)
(72) Erfinder: ROEMPP, Leo, 78739 Hardt (DE)
(74) Vertreter: Winter, Brandl - Partnerschaft mbB

(56) Entgegenhaltungen:
- EP-A1- 3 434 208
- EP-B1- 2 838 445
- US-A1- 2012 244 497
- US-A1- 2013 006 248
- US-A1- 2014 277 041
- US-A1- 2017 231 643
- US-B2- 7 892 235

## Beschreibung

### Technisches Gebiet

Die vorliegende Offenbarung betrifft ein chirurgisches Fräswerkzeug zur (rotierenden) fräsenden/ spanabhebenden Bearbeitung eines Gewebes, insbesondere eines Knochengewebes oder eines Knorpelgewebes. Das Fräswerkzeug weist einen proximalen Schaft/ Werkzeugschaft/ Spannabschnitt entlang einer Rotationsachse bzw. Längsachse des Fräswerkzeugs zur Ankopplung an eine Werkzeugaufnahme auf, und weist ferner einen distalen Fräskopf auf, an dessen Außenumfang Schneiden angeordnet sind, welche die Funktion des Fräsens ausführen.

### Hintergrund der vorliegenden Offenbarung

Rosenfräser sind allgemein als Standardfräser für allgemeine Fräsarbeiten am Knochen bekannt. Durch die Verwendung einer hohen Anzahl an Schneiden, üblicherweise ca. zehn Schneiden, ergibt sich ein relativ ruhiger Lauf bei einem bearbeitenden Eingriff in das zu zerspanende Gewebe, insbesondere in ein Knochengewebe. Diese hohe Anzahl an Schneiden bedeutet jedoch auch, dass sich das Fräswerkzeug meist überdurchschnittlich erwärmt und sich die geometrisch bedingt klein ausgebildeten Spannuten sehr schnell mit Gewebe zusetzen. Ferner laufen die Schneiden alle an der distalen Spitze des Fräswerkzeugs zusammen, weshalb die Spitze nicht schneidfreudig, sondern mehr stumpf ausgebildet ist. Außerdem gibt es bei einem Rosenfräser nach Stand der Technik einen (Übergangs-)Bereich zwischen einem hinterem/ proximalen Schneidenende und dem Werkzeugschaft des Fräswerkzeugs, der keine Schneidfunktion ausführt und daher zu einer erhöhten Wärmeentwicklung bei gleichzeitiger Einschränkung der möglichen Bearbeitungsrichtung fuhrt. Mit anderen Worten ist ein nach proximal ausgerichteter Bereich des Fräskopfs nicht für eine schneidende Bearbeitung ausgebildet und hemmt, wenn beispielsweise der Fräser seitlich verfahren wird, eine Art Rückziehen des Fräsers in Richtung der Rotationsachse aus dem Gewebe heraus.

Ein weiteres Beispiel eines Fräswerkzeugs nach Stand der Technik hat beispielsweise nur zwei Schneiden, wodurch zumindest größere Spannuten gebildet werden, welche sich zumindest nur sehr schwer zusetzen. Nachteile sind hierbei jedoch die für einen ruhigen Lauf benötigten und zusätzlich eingefügten Stützkanten, welche sich zwischen den einzelnen Schneiden befinden, um diese entsprechend zu stützen. Diese Stützkanten reiben dabei stumpf auf dem Werkstück und erzeugen eine unnötige und auch nachteilige Hitze. Ähnlich wie beim Rosenfräser beinhaltet also auch dieser zweischneidige Fräser einen stumpfen Bereich zwischen der Schneide und dem (Werkzeug-)Schaft des Fräswerkzeugs, welcher die gleichen vorstehend genannten Nachteile wie beim Rosenfräser hat. Dadurch, dass bei dem zweischneidigen Fräser nur zwei Schneiden zu der distalen Spitze zusammenlaufen, können zumindest diese Schneiden und auch die Spitze aggressiver bzw. schneidfreudiger (mit einer stärker ausgeprägten, spanabhebenden Schneidkante) als die des Rosenfräsers gestaltet werden. Diese aggressivere Spitze zentriert das Fräswerkzeug sehr gut, wodurch sich der zweischneidige Fräser hervorragend für bohrähnliche Arbeiten eignet, in welche in Längsrichtung in das Werkstück bzw. den zu bearbeitenden Knochen eingetaucht werden muss. Die vorstehend genannte Eigenschaft erschwert es jedoch, bei dem zweischneidigen Fräser aus der Funktion des Bohrens ausgehend in eine Funktion des Fräsens überzugehen und in eine spezielle Richtung quer zur Längsachse bzw. Rotationsachse mit einem Fräsen fortzufahren.

Beispielsweise ist ein gattungsgemäßes Schneidwerkzeug, das einen proximalen Werkzeugschaft und einen distalen Fräskopf aufweist, wobei der Fräskopf genau drei Schneiden am Außenumfang hat, welche sich spiralförmig mit einem veränderlichen Schneidenradius erstrecken, aus der US 2014/277041 A1, der US 2017/231643 A1, der US 2013/006248 A1, der EP 2 838 445 B1, der EP 3 434 208 A1, der US 7 892 235 B2 oder der US 2012/244297 A1 bekannt.

### Zusammenfassung der vorliegenden Offenbarung

Die Aufgabe der vorliegenden Offenbarung ist daher die Nachteile aus dem Stand der Technik zu vermeiden oder zumindest zu mindern und insbesondere ein chirurgisches Fräswerkzeug zur Verfügung zu stellen, welches einen flexiblen Einsatz gewährleistet und dabei sowohl ein Bohren zulässt als auch ein ("sanftes") Fräsen in alle Richtungen (insbesondere ohne harte Übergänge) ermöglicht, wobei eine Schneideffizienz erhöht wird, um eine möglichst geringe Wärmeentwicklung zu erzielen. Ferner ist eine Teilaufgabe, eine gute Abfuhr des "Gewebe-"Spans und einen ruhigen Lauf zu gewährleisten. Eine weitere Teilaufgabe ist eine Zentrierfunktion des Fräswerkzeugs bereitzustellen, um eine präzises Eintauchen in ein Gewebe zu ermöglichen.

Die Aufgaben der vorliegenden Offenbarung werden hinsichtlich eines gattungsgemäßen chirurgischen Fräswerkzeugs erfindungsgemäß durch die Merkmale des Anspruchs 1 gelöst. Vorteilhafte Weiterbildungen sind Gegenstand der abhängigen Ansprüche.

Ein Grundgedanke der vorliegenden Erfindung liegt also darin, ein chirurgisches Fräswerkzeug mit optimierter Geometrie bereitzustellen. Das Fräswerkzeug gemäß der vorliegenden Offenbarung (kann vorzugsweise auch als "Dreischneider" bezeichnet werden) zeichnet sich durch einen distalen Fräskopf aus, der drei Schneiden aufweist, insbesondere aus genau drei Schneiden und (den) dazwischenliegenden Freiwinkeln und Spannuten besteht. Die Kombination aus drei, insbesondere fast oder sogar direkt bis zu dem Werkzeugschaft durchgezogenen, Schneiden und einem positiven Drallwinkel (mit vorzugsweise entsprechenden Schneidwinkeln) sorgt dafür, dass die vorhandenen Freiflächen klein ausfallen und keine weiteren Stützflachen benötigt werden, wie dies etwa bei einem zweischneidigen Fräswerkzeug der Fall ist. Dadurch sind alle (potentiellen) Kontaktflächen des Fräswerkzeugs (mit dem Gewebe) schneidend ausgebildet, was in Folge zu einer sehr geringen Wärmeentwicklung führt (da insbesondere keine oder zumindest nur minimal Reibwärme erzeugt wird). Da eigentlich keine Kontaktfläche mehr eine stumpfe Reibung erzeugt, sondern die Kontaktflächen des Fräskopfs alle schneidend ausgebildet sind, kann eine Schneideffizienz des Fräswerkzeugs gesteigert werden. Aufgrund des positiven Drallwinkels können die Spannuten und auch die Schneiden vorzugsweise bis fast komplett oder sogar komplett an den Werkzeugschaft herangezogen werden, wodurch das Werkzeug in eigentlich alle Richtungen fräsen kann. Durch diese geometrische Konfiguration ergeben sich außerdem große Spannuten, die die Spanabfuhr des Fräswerkzeugs der vorliegenden Offenbarung begünstigen.

Mit anderen Worten wird also gemäß vorliegender Offenbarung ein chirurgisches Fräswerkzeug zur (rotierenden) fräsenden/ spanabhebenden Bearbeitung eines Gewebes, insbesondere eines Knochengewebes oder eines Knorpelgewebes, bereitgestellt mit: einem proximalen Werkzeugschaft/ Spannabschnitt entlang einer Rotationsachse/ Längsachse des Fräswerkzeugs zur Ankopplung an eine Werkzeugaufnahme, und einem distalen Fräskopf, an dessen Außenumfang Schneiden angeordnet sind. An dem Fräskopf sind dabei genau drei Schneiden angeordnet, wobei die Schneiden des Fräskopfs sich (in Richtung der Rotationsachse in proximaler Richtung gesehen) spiralförmig (beispielsweise in proximaler Richtung gesehen ähnlich einer archimedischen Spirale, wobei die Schneidspitzen der Schneiden die Spirale definieren) um die Rotationsachse mit veränderlichem Schneidenradius gegenüber der Rotationsachse erstrecken und einen positiven Drallwinkel oder eine positive Steigung aufweisen. Die Schneiden weisen ferner jeweils zwischen den Schneiden einen Freiwinkel sowie eine Spannut auf. Auch verringert sich vorzugsweise der Schneidenradius entlang der Rotationsachse von einem Maximum des Schneidenradius ausgehend in distaler Richtung hin, insbesondere kontinuierlich. Im Wesentlichen ist das Werkzeug entlang seiner Längsachse also in zwei Abschnitte unterteilt, einen proximalen Werkzeugschaft mit Funktion der Einspannung und einem proximalen Abschnitt zur Gewebebearbeitung.

Die Rotationsachse bzw. Längsachse des Fräswerkzeugs ist also eine gerade Achse, um welche sich das Fräswerkzeug, wenn in eine entsprechende Werkzeugaufnahme angekoppelt wie eingespannt, rotiert.

Der Schneidenradius wiederum wird von der Rotationsachse ausgehend in Richtung quer zu der Rotationsachse (bis zur radialen Schneidspitze) bestimmt. Wenn beispielsweise die Rotationsachse die X-Richtung definiert, so definiert der Schneidenradius die orthogonale Y-Richtung und folglich der Schneidradius die "Spitze" der Schneidkante in radialer Richtung. An dieser Stelle sei der Vollständigkeit halber angemerkt, dass an jeder Position der Rotationsachse ein Schneidenradius für die Schneiden nicht unbedingt gleich ausfallen muss. Beispielsweise kann an einer "X-Position" der Rotationsachse die erste Schneide einen ersten Schneidenradius aufweisen und die zweiten Schneiden einen hiervon unterschiedlichen Schneidenradius. Insbesondere weisen jedoch alle Schneiden (bis auf eventuell einen Bereich der distalen (Werkzeug-)Spitze), an jeder X-Position in Richtung der Rotationsachse einen gleichen Schneidenradius auf, um einen ruhigen Lauf zu gewährleisten.

Der Begriff "spiralförmig" meint vorliegend, dass bei einer Frontansicht von distal nach proximal in Richtung der Rotationsachse, die einzelne Schneide(-spitze) spiralförmig zumindest in Richtung der distalen Spitze zusammenläuft, wobei nicht zwingendermaßen eine gleichbleibende bzw. stetige Verkleinerung des Schneidenradius erfolgen muss, also die Form der Spirale nicht zwangsweise einen ähnlichen Abstand von einer Spiralebene zur nächsten Spiralebene hat. Insbesondere kann die Schneide auch nach proximal spiralförmig zusammenlaufen. Entscheidend hierbei ist also, dass der Fräskopf keinen konstanten Schneidenradius über die Rotationsachse aufweist, sondern dass die Schneiden einen veränderlichen Schneidenradius aufweisen. Insbesondere erhöht sich durch den Drallwinkel oder die Steigung eine Länge der Schneidenkante und optimiert ferner einen Schneidprozess. Insbesondere kann jedoch hierdurch die Schneidenkante bis kurz vor oder bis direkt zu dem Werkzeugschaft gezogen werden, so dass die radialen (und ferner auch potenzielle axiale) Kontaktflächen allesamt als Schneiden ausgebildet sind und nicht reiben und unnötig Wärme erzeugen.

Gemäß der Offenbarung erstreckt sich / verläuft eine (insbesondere einzige) Schneide der Schneiden des Fräskopfs (direkt) bis zu der Rotationsachse und bildet damit die distale Spitze mit einem distalen Schneidenradius von null, während die verbleibenden Schneiden als unterbrochene Schneiden ausgebildet sind und einen Abstand in Richtung der Rotationsachse zu der distalen Spitze sowie einen Schneidenradius von größer null aufweisen. Hierdurch bildet die eine Schneide, welche bis zu der Rotationsachse durchgezogen ist, die distale Spitze des Fräswerkzeugs. Insbesondere kann diese Schneide dann eine Art einzige Bohrspitze (Zentrierspitze) bilden, um für eine Bohrung in eine ebene Oberfläche hinein einen Auflagestützpunkt (bzw. Zentrierung) zu bilden und hiervon ausgehend den Radius nach und nach zu erweitern. Die Spitze des Fräswerkzeugs bildet daher nur eine der Schneiden, welche bis zu der Rotationsachse verläuft und dort spitz zulauft. Die anderen zwei Schneiden sind schon vorher (vor der Spitze) unterbrochen und verlaufen nicht bis zur Spitze, wodurch dort sehr viel Spanraum entsteht, was wiederum zu einer schneidfreudigen Werkzeugspitze führt. Die beschriebene Werkzeugspitze zentriert dieses beim Bohren oder Fräsen gerade hinreichend, um ein problemloses Eintauchen des Fräswerkzeugs in das Gewebe zu ermöglichen, hindert den Anwender jedoch nicht daran, die Vorschubrichtung zu wechseln. Daher kann mit dem dreischneidigen Fräswerkzeug frei in alle Richtungen gefräst werden, ohne dass der Anwender eine sogenannte "tote Stelle" überwinden muss.

Gemäß einer weiteren Ausführungsform kann der Abstand der unterbrochenen Schneiden (in Richtung der Rotationsachse; ähnlich der X-Richtung) zu der distalen Spitze jeweils gleich ausgebildet sein. Hierdurch wird durch alle Schneiden eine Ebene in Richtung der Rotationsachse gebildet. Alternativ kann vorzugsweise der Abstand der unterbrochenen Schneiden zu der distalen Spitze unterschiedlich zueinander ausgebildet sein, um in Richtung der Rotationsachse eine stufenförmige Erhöhung der Anzahl der Schneiden zu bewirken. Bei einem Eintauchen des Fräswerkzeugs in das Gewebe kommen somit nach und nach die Schneiden distal in Kontakt mit dem Gewebe. Mit anderen Worten kann Abstand der unterbrochenen Schneiden bzw. der nicht durchgezogenen Schneiden zur Spitze gleich oder (etwa leicht) versetzt ausgewählt werden.

Vorzugsweises kann sich der Spanwinkel und/oder der Freiwinkel der Schneide sich über den Verlauf der Schneide ändern (variieren), um ein optimales Fräsergebnis zu erzielen. Durch eine Unterteilung des Fräskopfs in Richtung der Rotationsachse in unterschiedliche Abschnitte mit jeweils zugewiesenen Spanwinkel bzw. Freiwinkel, kann für die jeweilige Behandlung eine optimale Konfiguration eingestellt werden. Wenn beispielsweise der Spanwinkel von distal nach proximal erhöht wird, so kann bei eine Materialabnehmung distal unterschiedlich gegenüber proximal eingestellt werden, sodass die geometrischen Strukturen (auch hinsichtlich einer mechanischen Belastbarkeit) optimal eingestellt werden können. Alternativ kann auch eine gleiche Spanabhebung realisiert werden. Mit anderen Worten können sich der Span- und/oder Freiwinkel über den Verlauf der Schneide ändern, insbesondere über den Verlauf aller drei Schneiden verändern, vorzugsweise einheitlich, so dass an jeder Position der Rotationsachse ein Span- bzw. Freiwinkel aller drei Schneiden (bis auf vorzugsweise die Spitze) gleich ist.

Gemäß einer weiteren Ausführungsform kann der Spanwinkel der (insbesondere genau einen) Schneide, die sich bis (direkt) zu der Rotationsachse erstreckt und damit die distale Spitze bildet, (direkt) an der distalen Spitze minimal -5° und/oder maximal +10° betragen, insbesondere genau 0° betragen. Diese Gestaltung ist besonders Vorteilhaft in Hinblick für ein problemloses Eintauchen (Bohren) in das Gewebe als auch einen effizienten Wechsel der Vorschubrichtung beim Fräsen.

Gemäß einer besonderen Ausführungsform kann der Spanwinkel der Schneiden in einem restlichen, von der Spitze beabstandeten Abschnitt minimal 0° und/oder maximal +15° betragen, insbesondere genau +10° betragen. Der Spanwinkel aller Schneiden ändert sich also in einem Bereich, der von der distalen Spitze in Richtung der Rotationsachse (proximal) beabstandet ist und liegt insbesondere in einem WinkelBereich von 0° bis +15°.

Vorzugsweise kann ein Freiwinkel der (insbesondere genau einen) Schneide, die sich bis zu der Rotationsachse erstreckt, an der distalen Spitze minimal +10° und/oder maximal +25° betragen, insbesondere genau 23° betragen. Auch diese geometrische Konfiguration der Schneide an der distalen Spitze ist vorteilhaft für eine hohe Effizienz und entsprechend gutes Schneidresultats.

Insbesondere kann ein Freiwinkel der Schneide, insbesondere aller drei Schneiden, in einem Abstand von 2mm bis 3mm, bevorzugt von 2,6 mm von der Spitze entfernt minimal +10° und/oder maximal +25° betragen, insbesondere genau 18° betragen.

Vorzugsweise kann ein durchgehender Freiwinkel der Schneide, welche bis zu der distalen Spitze durchgeht bzw. bis zu der Rotationsachse sich erstreckt, minimal +15° und/oder maximal +40° betragen, insbesondere genau 25° sein.

Gemäß einer Ausführungsform kann der Drallwinkel der Schneide (von dem proximalen Start der Schneide am Fräskopf im Bereich des Werkzeugschafts bis zu dem distalen Ende der Schneide (wobei zumindest eine Schneide die distale Spitze definiert), insbesondere aller Schneiden, minimal 15° und/oder maximal 20° betragen, insbesondere genau 17,4° betragen. Alternativ oder zusätzlich kann auch eine Steigung der Schneide mit minimal 40 mm und/oder maximal 80mm, insbesondere 60mm vorgesehen sein.

Gemäß einer weiteren Ausführungsform können die Schneiden entlang der Rotationsachse in proximaler Richtung bis an den Werkzeugschaft gezogen sein, insbesondere direkt in den Werkzeugschaft übergehen, oder zumindest bis zu einem Abstand von maximal 20%, bevorzugt von maximal 10% der Abmessung des Fräskopfs in Richtung der Rotationsachse an der Werkzeugschaft heranreichen bzw. von diesem beabstandet sein.

Insbesondere kann jede Schneide jeweils genau zwei Freiflächen aufweisen. Alternativ kann eine Schneide jeweils auch nur genau eine Freifläche aufweisen. Weiter alternativ kann eine Schneide auch mehrere Freiflächen aufweisen. Mit anderen Worten kann das Fräswerkzeug zwei Freiflachen pro Schneide beinhaltet, wobei alternativ auch mehr oder weniger Freiflächen möglich sind.

Insbesondere sind die drei Schneiden rotationssymmetrisch ausgebildet, bis auf möglicherweise den Abschnitt der distalen Spitze, in dem Fall, dass nur eine Schneide bis zu der Rotationsachse verläuft. Mit anderen Worten ist vorzugsweise der Fräskopf zumindest in einem proximalen Bereich, insbesondere insgesamt rotationssymmetrisch (mit drei gleichen Abschnitten) gestaltet.

Gemäß der Offenbarung erhöht sich ausgehend von dem Werkzeugschaft ein Schneidenradius in distaler Richtung entlang der Rotationsachse kontinuierlich/stetig bis zu einem maximalen Schneidenradius, und verringert sich hiernach dann kontinuierlich. Damit wird gewissermaßen eine Form einer Beere oder einer Rose nachempfunden.

Insbesondere kann der Scheidenradius an einer Position der Rotationsachse an allen Schneiden gleich sein, zumindest in einem Bereich des Fräskopfs außerhalb der Spitze (also bis auf eventuell die Spitze).

Das Fräswerkzeug kann insbesondere nach Art eines Rosenfräsers ausgebildet sein und insbesondere eine solche Außenkontur aufweisen.

Vorzugsweise kann ein Übergangsabschnitt an dem Fräskopf zwischen dem Werkzeugschaft und den Schneiden (in Richtung der Rotationsachse gesehen) vorgesehen sein, der vorzugsweise eine schalenförmige oder teilsphärische Außenoberfläche aufweist.

Insbesondere kann ein Durchmesser des Fräskopfs oder eine Schneidendurchmesser 6 mm betragen.

Vorzugsweise sind alle Kontaktflächen des Fräswerkzeugs, welche bei einem Eintauchen in das Gewebe mit dem Gewebe (zuerst) in Kontakt kommen sowie bei einem Fräsen mit dem Gewebe in Kontakt kommen schneidend ausgebildet. Hierdurch wird ein nachteiliges Reiben des Fräswerkzeugs mit dem Gewebe vermieden und es werden alle Kontaktflächen weisen nur Schneidkaten auf.

Insbesondere sind die Schneiden umfänglich gleichverteilt angeordnet.

Insbesondere kann das Fräswerkzeug die folgenden Maße bzw. Winkel des Fräskopfs aufweisen, wobei der Fräskopf ferner insbesondere einen Durchmesser von 6mm aufweist:

| **Maß** | Optimale Kombination | Minimum | Maximum |
|---|---|---|---|
| Spanwinkel γ (Spitze) | 0° | -5° | 10° |
| Spanwinkel γ (Rest der Schneide) | 10° | 0° | 15° |
| Freiwinkel α (Spitze) | 23° | 10° | 25° |
| Freiwinkel α (2,6mm von Spitze entfernt) | 18° | 10° | 25° |
| Freiwinkel α2 (durchgehend) | 25° | 15° | 40° |
| Drallwinkel β / Steigung | 17,4° / 60mm | - / 40mm | - / 80mm |

Die Spalte "optimale Kombination" beschreibt dabei die Werte, welche besonders optimale Werte bezüglich einer Effizienz des Fräswerkzeugs der vorliegenden Offenbarung aufweisen. Die Spalten "minimaler Wert bzw. maximaler Wert" definieren die Grenzen, innerhalb derer das Fräswerkzeug noch angemessen seine Funktion erfüllen kann.

Insbesondere beträgt ein Winkel einer Tangente der Schneide an der distalen Spitze gegenüber der Rotationsachse zwischen +60° und +80°, insbesondere +70°.

### Kurzbeschreibung der Figuren

Die vorliegende Offenbarung wird nachfolgend anhand einer bevorzugter Ausführungsform mit Hilfe von begleitenden Figuren näher erläutert. Es zeigen:
- Fig. 1: eine Seitenansicht eines chirurgischen Fräswerkzeugs gemäß einer bevorzugten Ausführungsform der vorliegenden Offenbarung;
- Fig. 2: eine Querschnittsansicht A - A durch den Fräskopf des Fräswerkzeugs aus Fig. 1;
- Fig. 3: eine detaillierte Teilansicht des Fräskopfs des Fräswerkzeugs aus Fign. 1 und 2; und
- Fig. 4: eine isometrische Ansicht des chirurgischen Fräswerkzeugs aus Fig. 1 bis 3.

Die Figuren sind schematischer Natur und sollen nur dem Verständnis der Erfindung dienen. Gleiche Elemente sind mit denselben Bezugszeichen versehen.

### Detaillierte Beschreibung bevorzugter Ausführungsformen

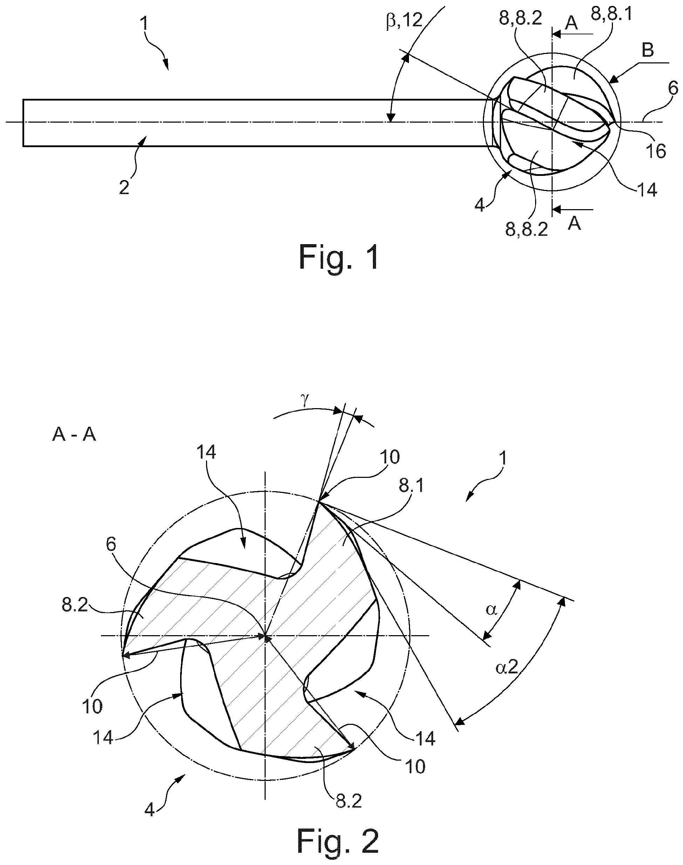

Fign. 1 bis 4 zeigen in verschiedenen Ansichten eine bevorzugte Ausführungsform eines chirurgischen Fräswerkzeugs 1 (nachstehend nur Fräswerkzeug genannt) gemäß der vorliegenden Offenbarung.

Das Fräswerkzeug 1 (als Rotationswerkzeug) der vorliegenden Ausführungsform dient zur fräsenden Bearbeitung eines Knochengewebes. Das Fräswerkzeug 1 ist im Wesentlichen in zwei Funktionsabschnitte unterteilt, nämlich einerseits einen proximalen (Werkzeug-)Schaft 4 (nachstehend nur Schaft bezeichnet) und andererseits eine distalen Fräskopf 4. Vorliegend werden die Bezeichnungen "proximal" und "distal" in Bezug auf eine Rotationsachse 6 des Fräswerkzeugs 1 verwendet und in Bezug auf einen Anwender gesehen, der das Werkzeug entsprechend bedient, wobei der distale Fräskopf 4 von dem Anwender abgewandt und entsprechend distal zu ihm angeordnet ist.

Der proximalen Werkzeugschaft 2 entlang der Rotationsachse 6 bzw. Längsachse des Fräswerkzeugs 1 dient der Funktion einer Ankopplung des Fräswerkzeugs an eine Werkzeugaufnahme und ist in dieser Ausführungsform zylinderförmig ausgeführt, um in ein Spannsystem eingespannt zu werden, beispielsweise eines Spannsystems eines Handstücks.

Der distalen Fräskopf 4 wiederum dient der Funktion einer fräsenden Bearbeitung und weist an seinem (radialen) Außenumfang entsprechende Schneiden 8 auf, die um die Rotationsachse 6 rotieren.

Im Gegensatz zum Stand der Technik weist der Fräskopf eine vorbestimmte Anzahl an Schneiden 8 auf, nämlich genau drei Schneiden 8. Diese Anzahl von drei Schneiden 8 ist der optimale Kompromiss zwischen einem ruhigen Vorlauf eines Fräsens und andererseits einer effizienten Schneidenfunktion bei nur sehr geringer Wärmeentwicklung, wie auch noch nachstehend detailliert erläutert.

Die Schneiden 8 des Fräskopfs 1 winden sich gewissermaßen spiralförmig um die Rotationsachse 6 in distaler Richtung und haben eine veränderlichen Schneidenradius 10 gegenüber der Rotationsachse 6. Dabei weisen die Schneiden 8 ferner einen positiven Drallwinkel β oder eine positive Steigung 12 auf. Hierdurch haben die Schneiden 8 jeweils zwischen den Schneiden 8 einen Freiwinkel α; α2 sowie eine Spannut 1, um das abgefräste Knochengewebe entsprechend abzutransportieren. Man kann auch sagen, dass von einer Frontansicht in proximale Richtung hin die Schneidkanten (also die Spitzen der Schneiden) sich spiralförmig mit sich verringerndem Schneidenradius 10 um die Rotationsachse 6 erstrecken.

Ferner verringert sich der der Schneidenradius 10 sich entlang der Rotationsachse 6 von einem Maximum des Schneidenradius 10 ausgehend in distaler Richtung hin kontinuierlich. Dadurch bildet sich bei dem Fräskopf 4 eine Art Rosenform oder eine Art Haselnussform aus. Man kann auch sagen, dass das Fräswerkzeug nach Art eines Rosenfräsers gestaltet ist, wobei die Anzahl der Schneiden auf drei Schneiden 8 reduziert ist und zudem, wie noch nachstehend erläutert, eine distale Spitze 16 bzw. der Abschnitt der distalen Spitze speziell gestaltet ist. Diese geometrische Gestaltung sorgt dafür, dass die vorhandenen Freiflächen klein ausfallen und keine weiteren Stützflächen benötigt werden. In Folge können alle Kontaktflächen des Fräswerkzeugs 1, welches mit dem Gewebe (als erstes) in Kontakt kommt schneidend gestaltet werden.

Von den drei Schneiden 8 sind zwei Schneiden 8.2 unterschiedlich zu einer (ersten) Schneide 8.1 ausgebildet. Konkret erstreckt sich eine (durchgehende) Schneide 8.1 der Schneiden 8 bis hin zu der Rotationsachse 8 und bildet damit die distale Spitze 16 mit einem distalen Schneidenradius 10 von null. Aufgelegt auf ein Knochengewebe kann diese distale Spitze als Zentrierung für ein Eintauchen in das Knochengewebe dienen. Die anderen verbleibenden Schneiden sind als unterbrochene Schneiden 8.2 ausgebildet und enden in Richtung der Rotationsachse gesehen in einem Abstand 18 zu der distalen Spitze 16. Ferner weisen diese unterbrochenen Schneiden 8.2 auch in ihrem distalen Bereich noch einen Schneidenradius 10 von größer null auf. Hierdurch kann im Bereich der distalen Spitze 16 (welche auf der Rotationsachse 6 liegt) auch ein großer Spanraum vorgesehen werden. Der Abstand 18 der unterbrochenen Schneiden 8.2 zu der distalen Spitze 16 ist in dieser Ausführungsform jeweils gleich ausgebildet.

Ferner ändert sich ein Spanwinkel γ sowie ferner der Freiwinkel α; α2 aller drei Schneiden 8 (bzw. der radialen Schneidkanten) über den Verlauf der Schneiden 8.

In dieser speziellen Ausführungsform beträgt der Spanwinkel γ an der Spitze 16 der Schneide 8.1 0° während er dann (nach proximal) für den Rest der Schneide(n) 8 außerhalb der Spitze sich auf +10° erhöht.

Der Freiwinkel α an der distalen Spitze 16 beträgt hier +23° und verringert sich in einem Abstand von 2,6mm zu der distalen Spitze zu einem Freiwinkel α von 18°.

Der durchgehende Freiwinkel α2 beträgt hier 25° und der Drallwinkel β beträgt 17,4°.

Die distale Schneidkante (bzw. deren distale Tangente) der (durchgehenden) Schneide 8.1 weist einen Winkel von 70° gegenüber der Rotationsachse 6 bzw. einen Winkel zwischen sich und der Rotationsachse 6 von 70° auf.

Zudem hat jede einzelne Schneide 8 genau zwei Freiflächen.

Die Schneiden 8 sind in einem proximalen Abschnitt des Fräskopfs 4 gleich gestaltet (bzw. rotationssymmetrisch) und gehen so gut wie direkt in den Werkzeugschaft 2 über, um selbst in einem proximalen Bereich noch eine Schneidfunktion bereitzustellen und bei einem Vorschub in ein Material hinein sogar auch in einer Richtung der Rotationsachse 6 wieder herausfräsend zurückgezogen werden zu können.

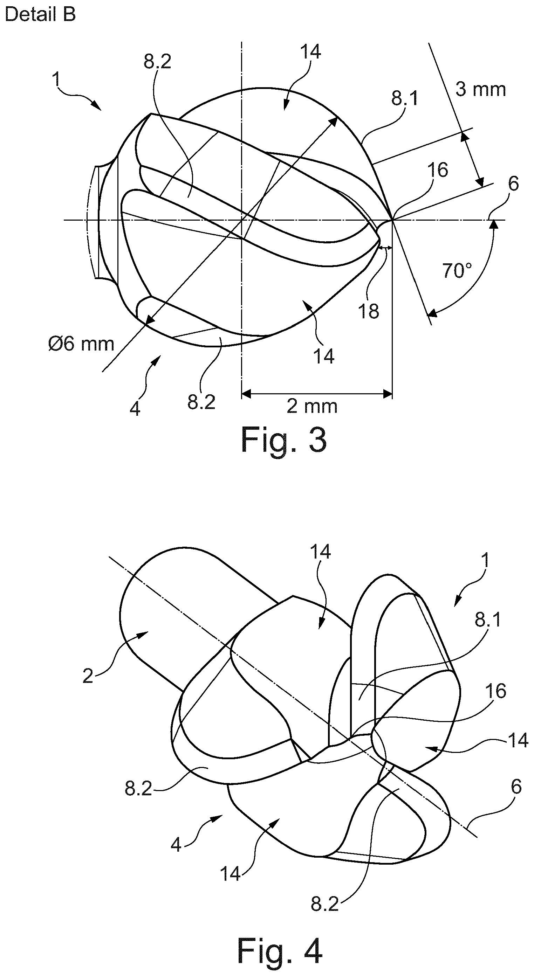

Wie insbesondere in Fig. 3 ersichtlich ändert sich ein Schneidenradius 10 (von der Rotationsachse 6 aus gesehen) kurvenförmig, wobei in einem proximalen Abschnitt des Fräskopfs 4 zunächst der Schneidenradius sich bis zu einem maximalen Schneidenradius 10 erhöht (etwa mittig in Richtung der Rotationsachse 6) um sich hiernach wieder kontinuierlich zu verringern.

Die Schneiden 8 weisen eine dreieckförmige Schneidkante auf, welche sich um die Rotationsachse 6 mit positivem Drall β windet.

Insbesondere können die Schneidkanten noch speziell gehärtet sein, um eine möglichst beständige und scharfe Schneide 8 bereitzustellen.

Der Werkzeugschaft 2 weist in dieser Ausführungsform eine zylinderförmige Außenkontur auf, wobei natürlich auch eine Nut in Richtung der Rotationsachse 6 in dem Werkzeugschaft vorgesehen werden kann, um formschlüssig sowohl eine axiale Fixierung als auch eine bessere Übertragung eines Drehmoments zu realisieren.

### Bezugszeichenliste:

- 1: Fräswerkzeug
- 2: Werkzeugschaft
- 4: Fräskopf
- 6: Rotationsachse
- 8: Schneiden
- 8.1: Schneide distale Spitze
- 8.2: Unterbrochene Schneide
- 10: Schneidenradius
- 12: Steigung
- 14: Spannut
- 16: Distale Spitze
- 18: Abstand (in Richtung Rotationsachse zur distalen Spitze)

- α: Freiwinkel
- α2: Freiwinkel (durchgehend)
- γ: Spanwinkel
- β: Drallwinkel

## Patentansprüche

1. Chirurgisches Fräswerkzeug (1) zur fräsenden Bearbeitung eines Gewebes, insbesondere eines Knochengewebes oder eines Knorpelgewebes, mit:
einem proximalen Werkzeugschaft (2) entlang einer Rotationsachse (6) des Fräswerkzeugs (1) zur Ankopplung an eine Werkzeugaufnahme, und
einem distalen Fräskopf (4), an dessen Außenumfang genau drei Schneiden (8) angeordnet sind,
wobei sich die Schneiden (8) des Fräskopfs (1) spiralförmig um die Rotationsachse (6) mit einem veränderlichen Schneidenradius (10) gegenüber der Rotationsachse (6) erstrecken, einen positiven Drallwinkel (β) oder eine positive Steigung (12) aufweisen, und jeweils zwischen den Schneiden (8) einen Freiwinkel (α; α2) sowie eine Spannut (14) aufweisen,
wobei
eine Schneide (8.1) der Schneiden sich bis zu der Rotationsachse erstreckt und damit die distale Spitze (16) mit einem distalen Schneidenradius (10) von null bildet, während die verbleibenden Schneiden als unterbrochene Schneiden (8.2) ausgebildet sind und einen Abstand (18) in Richtung der Rotationsachse (6) zu der distalen Spitze (16) sowie einen Schneidenradius (10) von größer null aufweisen, und
sich der Schneidenradius (10) ausgehend von dem Werkzeugschaft (2) in distaler Richtung entlang der Rotationsachse (6) kontinuierlich bis zu einem Maximum des Schneidenradius (10) erhöht und hiernach kontinuierlich verringert, um eine Art bauchförmige radiale Außenkontur zu bilden.

2. Chirurgisches Fräswerkzeug (1) nach Anspruch 1, **dadurch gekennzeichnet, dass**
der Abstand (18) der unterbrochenen Schneiden (8.2) zu der distalen Spitze (16) in Richtung der Rotationsachse (6) jeweils gleich ausgebildet ist, oder
der Abstand der unterbrochenen Schneiden (8.2) zu der distalen Spitze (16) unterschiedlich zueinander ausgebildet ist, um eine stufenförmige Erhöhung der Anzahl der Schneiden (8) in Richtung der Rotationsachse (6) zu bewirken.

3. Chirurgisches Fräswerkzeug (1) nach einem der vorstehenden Ansprüche, **dadurch gekennzeichnet, dass** ein Spanwinkel (γ) und/oder der Freiwinkel (α; α2) der Schneide (8) sich über den Verlauf der Schneide ändert.

4. Chirurgisches Fräswerkzeug (1) nach einem der vorstehenden Ansprüche, **dadurch gekennzeichnet, dass** der Spanwinkel (γ) der Schneide (8.1), die sich bis zu der Rotationsachse (6) erstreckt, an der distalen Spitze (16) minimal -5° und/oder maximal +10° beträgt, insbesondere genau 0° ist.

5. Chirurgisches Fräswerkzeug (1) nach Anspruch 4, **dadurch gekennzeichnet, dass** der Spanwinkel (γ) der Schneiden (8) in einem restlichen, von der Spitze beabstandeten Abschnitt minimal 0° und/oder maximal +15° beträgt, insbesondere genau +10° ist.

6. Chirurgisches Fräswerkzeug (1) nach einem der vorstehenden Ansprüche, **dadurch gekennzeichnet, dass** ein Freiwinkel (α) der Schneide (8.1), die sich bis zu der Rotationsachse (6) erstreckt, an der distalen Spitze (16) minimal +10° und/oder maximal +25° beträgt, insbesondere genau +23° ist.

7. Chirurgisches Fräswerkzeug (1) nach einem der vorstehenden Ansprüche, **dadurch gekennzeichnet, dass** ein Freiwinkel (α) der Schneide (8) in einem Abstand (18) von minimal 2mm und/oder maximal 3mm, insbesondere bei 2,6mm von der Spitze (16) entfernt minimal +10° und/oder maximal +25° beträgt, insbesondere genau +18° ist.

8. Chirurgisches Fräswerkzeug (1) nach einem der vorstehenden Ansprüche, **dadurch gekennzeichnet, dass** ein durchgehender Freiwinkel (α2) minimal +15° und/oder maximal +40° beträgt, insbesondere genau +25° ist.

9. Chirurgisches Fräswerkzeug (1) nach einem der vorstehenden Ansprüche, **dadurch gekennzeichnet, dass**
der Drallwinkel (β) der Schneide (8) minimal +15° und/oder maximal +20° beträgt, insbesondere +17,4° ist, und/oder
eine Steigung (12) der Schneide (8) minimal 40 mm und/oder maximal 80mm ist, insbesondere 60mm beträgt.

10. Chirurgisches Fräswerkzeug (1) nach einem der vorstehenden Ansprüche, **dadurch gekennzeichnet, dass** die Schneiden (8) entlang der Rotationsachse (6) in proximaler Richtung bis an den Werkzeugschaft (2) gezogen sind, insbesondere direkt in den Werkzeugschaft (2) übergehen.

11. Chirurgisches Fräswerkzeug (1) nach einem der vorstehenden Ansprüche, **dadurch gekennzeichnet, dass** jede Schneide (8) zwei Freiflächen aufweist.

## Claims

1. A surgical milling tool (1) for milling a tissue, in particular a bone tissue or a cartilage tissue, with:
a proximal tool shaft (2) along a rotational axis (6) of the milling tool (1) for coupling to a tool receptacle, and
a distal milling head (4), on the outer circumference of which exactly three blades (8) are arranged,
wherein the blades (8) of the milling head (1) extend spiral-shaped around the rotational axis (6) with a variable blade radius (10) relative to the rotational axis (6), have a positive angle of twist (β) or a positive pitch (12), and each have a relief angle (α; α2) and a chip flute (14) between the blades (8),
wherein
one blade (8.1) of the blades extends up to the rotational axis and thus forms the distal tip (16) with a distal blade radius (10) of zero, while the remaining blades are configured as discontinuous blades (8.2) and have a distance (18) in the direction of the rotational axis (6) to the distal tip (16) and a blade radius (10) of greater than zero, and
starting from the tool shaft (2), a blade radius (10) increases continuously in the distal direction along the rotational axis (6) up to a maximum of the blade radius (10) and then decreases continuously thereafter in order to form a kind of belly-shaped radial outer contour.

2. The surgical milling tool (1) according to claim 1, **characterized in that**
the distance (18) of the discontinuous blades (8.2) to the distal tip (16) in the direction of the rotational axis (6) is the same in each case, or
the distance of the discontinuous blades (8.2) to the distal tip (16) is different in order to achieve a stepped increase in the number of blades (8) in the direction of the rotational axis (6).

3. The surgical milling tool (1) according to one of the preceding claims, **characterized in that** a rake angle (γ) and/or the relief angle (α; α2) of the blade (8) changes over the course of the blade.

4. The surgical milling tool (1) according to one of the preceding claims, **characterized in that** the rake angle (γ) of the blade (8.1), which extends up to the rotational axis (6), at the distal tip is a minimum of -5° and/or a maximum of +10° (16), in particular is exactly 0°.

5. The surgical milling tool (1) according to claim 4, **characterized in that** the rake angle (γ) of the blades (8) in a remaining portion spaced from the tip is a minimum of 0° and/or a maximum of +15°, in particular is exactly +10°.

6. The surgical milling tool (1) according to one of the preceding claims, **characterized in that** a relief angle (α) of the blade (8.1), which extends up to the rotational axis (6), is a minimum of +10° and/or a maximum of +25° at the distal tip (16), in particular is exactly +23°.

7. The surgical milling tool (1) according to one of the preceding claims, **characterized in that** a relief angle (α) of the blade (8) at a distance (18) of minimum 2 mm and/or maximum 3 mm, in particular at 2.6 mm from the tip (16), is a minimum of +10° and/or a maximum of +25°, in particular is exactly +18°.

8. The surgical milling tool (1) according to one of the preceding claims, **characterized in that** a continuous relief angle (α2) is a minimum of +15° and/or a maximum of +40°, in particular is exactly +25°.

9. The surgical milling tool (1) according to one of the preceding claims, **characterized in that**
the angle of twist (β) of the blade (8) is a minimum of +15° and/or a maximum of +20°, in particular is +17.4°, and/or
a pitch (12) of the blade (8) is a minimum of 40 mm and/or a maximum of 80 mm, in particular is 60 mm.

10. The surgical milling tool (1) according to one of the preceding claims, **characterized in that** the blades (8) are drawn along the rotational axis (6) in the proximal direction up to the tool shaft (2), in particular merge directly into the tool shaft (2).

11. The surgical milling tool (1) according to one of the preceding claims, **characterized in that** each blade (8) has two clearance surfaces.

## Revendications

1. Outil de fraisage chirurgical (1) pour l'usinage par fraisage d'un tissu, en particulier d'un tissu osseux ou d'un tissu cartilagineux avec :
une tige d'outil (2) proximale le long d'un axe de rotation (6) de l'outil de fraisage (1) pour le couplage avec un logement d'outil et
une tête de fraisage (4) distale, au niveau de la périphérie extérieure de laquelle sont agencées trois lames (8),
dans lequel les lames (8) de la tête de fraisage (1) s'étendent en spirale autour de l'axe de rotation (6) avec un rayon de lame (10) modifiable par rapport à l'axe de rotation (6), présentent un angle de torsion positif (β) ou une pente (12) positive, et présentent respectivement entre les lames (8) un angle de dépouille (α ; α2) ainsi qu'une rainure à copeaux (14),
dans lequel
une lame (8.1) des lames s'étend jusqu'à l'axe de rotation et forme ainsi la pointe distale (16) avec un rayon de lame (10) distal de zéro, tandis que les lames restantes sont configurées comme lames (8.2) interrompues et présentent une distance (18) en direction de l'axe de rotation (6) par rapport à la pointe distale (16) ainsi qu'un rayon de lame (10) supérieur à zéro et
le rayon de lame (10) augmente en continu à partir de la tige d'outil (2) dans le sens distal le long de l'axe de rotation (6) jusqu'à un maximum du rayon de lame (10) et diminue ensuite en continu afin de former un type de contour extérieur radial convexe.

2. Outil de fraisage chirurgical (1) selon la revendication 1, **caractérisé en ce que**
la distance (18) des lames (8.2) interrompues par rapport à la pointe distale (16) dans le sens de l'axe de rotation (6) est formée respectivement de manière identique ou
la distance des lames interrompues (8.2) par rapport à la pointe distale (16) est formée de manière différente l'une à l'autre afin de provoquer une augmentation étagée du nombre de lames (8) dans le sens de l'axe de rotation (6).

3. Outil de fraisage chirurgical (1) selon l'une quelconque des revendications précédentes, **caractérisé en ce qu'**un angle de coupe orthogonal (γ) et/ou l'angle de dépouille (α ; α2) de la lame (8) se modifie sur l'étendue de la lame.

4. Outil de fraisage chirurgical (1) selon l'une quelconque des revendications précédentes, **caractérisé en ce que** l'angle de coupe orthogonal (γ) de la lame (8.1) qui s'étend jusqu'à l'axe de rotation (6), s'élève au niveau de la pointe distale (16) au minimum à -5° et/ou au maximum à +10°, en particulier est précisément de 0°.

5. Outil de fraisage chirurgical (1) selon la revendication 4, **caractérisé en ce que** l'angle de coupe orthogonal (γ) des lames (8) s'élève dans une section restante espacée de la pointe au minimum à 0° et/ou au maximum à +15°, en particulier est précisément de +10°.

6. Outil de fraisage chirurgical (1) selon l'une quelconque des revendications précédentes, **caractérisé en ce qu'**un angle de dépouille (α) de la lame (8.1) qui s'étend jusqu'à l'axe de rotation (6), s'élève au niveau de la pointe distale (16) au minimum à +10° et/ou au maximum à +25°, en particulier est précisément de +23°.

7. Outil de fraisage chirurgical (1) selon l'une quelconque des revendications précédentes, **caractérisé en ce qu'**un angle de dépouille (α) de la lame (8) est à une distance (18) de 2 mm au minimum et/ou de 3 mm au maximum, s'élève en particulier en cas de distance de 2,6 mm de la pointe (16), à +10° au minimum et/ou +25° au maximum, est en particulier précisément de +18°.

8. Outil de fraisage chirurgical (1) selon l'une quelconque des revendications précédentes, **caractérisé en ce qu'**un angle de dépouille (α2) continu s'élève au minimum à +15° et/ou au maximum à +40°, en particulier est précisément de +25°.

9. Outil de fraisage chirurgical (1) selon l'une quelconque des revendications précédentes, **caractérisé en ce que**
l'angle de torsion (β) de la lame s'élève à +15° au minimum et/ou +20° au maximum, en particulier est de +17,4°, et/ou
une pente (12) de la lame (8) est au minimum de 40 mm et/ou au maximum de 80 mm, en particulier s'élève à 60 mm.

10. Outil de fraisage chirurgical (1) selon l'une quelconque des revendications précédentes, **caractérisé en ce que** les lames (8) sont tirées le long de l'axe de rotation (6) dans le sens proximal jusqu'à la tige d'outil (2), passent en particulier directement dans la tige d'outil (2).

11. Outil de fraisage chirurgical (1) selon l'une quelconque des revendications précédentes, **caractérisé en ce** quechaque lame (8) présente deux surfaces de dépouille.
